# EUROPEAN PATENT APPLICATION

(11) **EP 1 133 993 A1**
(43) Date of publication of application: **19.09.2001**
(21) Application number: 00105081.4
(22) Date of filing: 10.03.2000
(51) Int. Cl.: A61K 38/17, A61K 48/00, A61K 31/56, C12N 5/10, G01N 33/50

(54) **Substances for the treatment of spinal muscular atrophy**

(71) Applicant: Max-Planck-Gesellschaft zur Förderung der Wissenschaften e.V., 80539 München (DE); Wirth, Brunhilde, 53225 Bonn (DE); Hofmann, Yvonne, 53225 Bonn (DE)
(72) Inventor: Stamm, Stefan, 81375 München (DE); Wirth, Brunhilde, 53225 Bonn (DE); Hofmann, Yvonne, 53225 Bonn (DE); Androphy, Elliot, Natick, MA 01760 (US); Lorson, Chris, Somerville, MA 02144 (US)
(74) Representative: Oser, Andreas

(57) **Abstract**

Substances which are capable of preventing the skipping or exclusion of exon 7 of the SMN gene are described. The substances can reverse "wrong" SMN2 splicing pattern, thus generating full length transcripts and thus functionally active SMN protein on the basis of the SMN2 gene *in vivo*. Since "wrong" SMN2 splicing is associated with disorder or disease states causing spinal muscular atrophy, but SMN2 genes still exist in viably individuals, the substances can be used as therapeutic agents. The invention also provides a method for testing substances that may control exon 7 inclusion of the SMN2 gene. The method is suitable for a high throughput screening.

## Description

The present invention relates to substances which are suitable for the treatment of spinal muscular atrophy, and to a method of testing the effectiveness of such substances for being suitable for the treatment of spinal muscular atrophy, along with products being useful therefor.

Spinal muscular atrophy (SMA) is a neurodegenerative disorder with progressive paralysis caused by the loss of alpha-motor neurons in the spinal cord. The disease is accompanied with a progressive weakness of the limbs and trunk, followed by muscle atrophy. SMA patients are subdivided in type I-III according to age of onset and achieved motor abilities (Munsat, T.L., and Davies, K.E., International SMA consortium meeting, 26-28 June 1992, Bonn, Germany, *Neuromusc*. *Disord.* 2, 423-428 (1992)). Werdnig-Hoffman disease, or type I SMA, is the most severe form, with onset either in utero or during the first few months of life. Affected children cannot sit unsupported, and death usually occurs up to two years of age. Type II SMA usually manifests within the first year of life, and, although affected children may sit unaided, they do not achieve the ability to stand or walk independently. The survival of type II individuals depends on the degree of respiratory complications. Kugelberg-Welander disease, or type III SMA, is a less severe form, characterized by a later age of onset. Affected individuals have variable severity, walk independently, and may have a normal life expectancy. SMA occurs in approximately 1 in 10,000 live births and has a carrier frenquency of 1 in 50. SMA thus represents the second most frequent autosomal recessive disorder. Further information about the molecular genetics and its implication for disease process and clinical phenotype is provided by A.H.M. Burghes, Am. J. Hum. Genet. 61, 9-15 (1997), and J. Melki, Curr. Opin. Neurol. 10, 381-385 (1997).

There are two copies of the *survival motor neuron gene (SMN)*, i.e. the SMA determining gene, present on chromosome 5q13, telomeric *(SMN1)* and centromeric *(SMN2)*, although they are not directly adjacent to each other. There are limited DNA sequence divergence between the two copies, but their predicted protein products are identical at the amino acid level. More than 96% of all SMA patients show homozygous loss of the *SMN1* gene caused by deletions or gene conversions (Lefebvre, S. et al. Cell 80, 155-165 (1995), Wirth, B. Hum Mutation, in press). While SMA is caused by homozygous loss of *SMN1*, homozygous absence of *SMN2* has no phenotypic effect. Specifically, while *SMN1* produces only full-length SMN transcript (FL-SMN mRNA), *SMN2* expresses dramatically reduced full-length and abundant levels of transcript lacking exon 7 (SMN2Δ7), encoding for a less stable protein that has a reduced self-oligomerization capacity (Lorson, C. L. and Androphy, E. J., Hum. Mol. Genet. 9, 259-265 (2000); Lorson, C.L. et al., Nature Genet. 19, 63-66 (1998); Pelizzoni, L., et al., Proc. Natl. Acad. Sci. USA. 96:11167-11172 (1999)). Therefore, maintenance and expression of *SMN2* does not overcome the loss of *SMN1* and cannot provide protection from disease development, despite the fact that both genes encode identical proteins. While the *SMN1* gene is lacking, an increasing copy number of the *SMN2* gene correlates with a decrease of the severity of the SMA form. At least a single copy of *SMN2* is required to maintain viability and is therefore present in all SMA patients.

The molecular cause of the difference in the splicing result between *SMN1* and *SMN2* has been identified recently by C.L. Lorson et al. (Proc. Nat. Acad. Sci. USA 96, 6307-6311 (1999); Lorson, C. L. and Androphy, E. J., Hum. Mol. Genet. 9, 259-265(2000)). The coding region of the two genes differs by a translationally silent nucleotide exchange in exon 7 (a C-to-T transition in position +6 of exon 7) that disrupts an exonic splicing enhancer (ESE) and causes exon 7 skipping in *SMN2* transcripts (SMN2Δ7). Based on this finding of the molecular basis, the diagnosis of SMA is straightforward, for example by means of PCR analysis being directed to the decisive *SMN1* exon 7 deletion.

Unfortunately, a therapy for the treatment of SMA does not exist yet. Based on the finding of the molecular basis for the occurrence of SMA, C.L. Lorson at al. (1999), *supra,* propose to make future efforts in gene therapy targeting on the single nucleotide conversion at the exon 7+6 position, thereby functionally converting *SMN2* into *SMN1*.

It is an object of the present invention to provide a substance which can be effective as a therapeutic agent for the treatment of spinal muscular atrophy (SMA). A further object of the present invention is to provide a process which is suitable for modifying the SMN isoform pattern of a mammalian cell.

Another object of the present invention is to provide a method which is suitable for a high throughput screen for substances that can be effective for the treatment of SMA, along with products which are useful for such a screening method.

Accordingly, the present invention provides a substance which is capable of preventing the skipping (exclusion) of exon 7 of the *SMN2* gene. Thus, the substance is suitable for the use as a therapeutic agent.

The present invention also provides a process for changing the pre-mRNA processing relating to the SMN gene of a mammalian cell, which process comprises exposing the cell to a substance, such as the afore-mentioned substance, which is capable of controlling the inclusion of exon 7 of the *SMN2* gene.

The present invention also provides a mammalian host cell which is stably transfected with a DNA encoding a polypeptide which is capable of at least partially preventing the skipping (exclusion) of exon 7 of the *SMN2* gene. The transfected mammalian host cell, which preferably originates from a human individual to be treated and/or from a cultured human cell or cell line, is useful in gene therapy.

The present invention also makes available a useful, high throughput screening method for the testing of substances that prevent the skipping (exclusion) of exon 7 of the *SMN2* gene, along with useful products being particularly suitable for such a screening method.

The present invention will now be described in further detail while referring to the accompanying figures, where:

Fig. 1 shows the sequence, on the RNA level, of the exon 7 and parts of adjacent introns 6 and 7 of the human *SMN1* gene, whereas the corresponding portion of the *SMN2* sequence is defined by a C-to-T transition at position +6 of exon 7 (identified by an asterisk).

Fig. 2 shows a schematic representation of a *SMN* exon 7 containing minigene expression construct based on *SMN2* wildtype used according to the present invention, wherein the sequence differences to *SMN1* are indicated.

Figs. 3A and 3B show schematic representations of vectors which respectively contain a DNA sequence encoding for a splicing regulator for *SMN2* exon 7.

Figs. 4A and 4B show the effects of a substance according to the present invention on the prevention of *SMN2* exon 7 skipping *in vivo,* wherein Fig. 4A shows the effects in human cells whereas Fig. 4B shows the effects in mouse cells.

Fig. 5 shows the *SMN2* exon 7 skipping effects after exposing human cells, which have been transfected by various SMN minigene constructs, to a DNA substance according to the present invention by means of co-transfection.

Fig. 6 shows the effects of another substance affecting *SMN2* exon 7 skipping *in vivo.*

According to the present invention, it was surprisingly found that the disease-causing, "wrong" splicing event, which mainly produces an aberrant SMN product, can be reversed *in vivo* by the use of certain substances which are capable of preventing the skipping (exclusion) of exon 7 of the *SMN2* gene. Thus, the substance according to the present invention enables the re-instatement of the functionally effective *SMN* protein on the basis of forming the full length *SMN* transcript including the *SMN* exon 7 sequences. Typically, only *SMN1* produces sufficient levels of *SMN* protein to provide protection from disease, however all viable SMA patients retain at least a single copy of *SMN2*. Thus, the therapeutic concept of the present invention is based on the compensation of the loss or misfunction of the SMA-determining gene *SMN1* by the generation of a full-length *SMN2* transcript which is made possible by the prevention or reversal of exon 7 skipping of the *SMN2* gene in the *in vivo* situation by means of the substance of the present invention. Since the *SMN2*-derived full length transcript encodes the corresponding active SMN protein, the substance according to the present invention can be effective as a therapeutic agent, especially for treating SMA. The desired effect can be attained if the exon 7 skipping of the *SMN2* gene is prevented or reversed partially or totally.

On account of the individual to be treated, the SMN gene and, correspondingly, its exon 7 to be controlled is preferably of human origin. However, the genetic source of the SMN gene is not limited to humans, because the individual to be treated is not limited to humans, but other species, especially mammals, can be treated as well. For example, since an SMA transgenic mouse exists in which the human *SMN2* gene has replaced the murine *Smn* gene (Hsieh-Li HM, Chang JG, Jong YJ et al. Nature Genet 24, 66-70 (2000), and Monani et al., Hum. Mol. Genet. 9, 333-339 (2000)), such a mouse model could be used for the therapeutic treatment to confirm the effectiveness of the therapeutic substance. The total human *SMN* gene can be derived from the website http://www.ncbi.nlm.nih.gov, wherein the cDNA relating to *SMN1* is listed under Genebank accession No. U18423 or A77035, and the cDNA relating to *SMN2* is listed under accession No. A77033. The sequence of *SMN1* exon 7 is shown under accession No. U43883, together with *SMN1* exon 8. Exon 7 of *SMN2* exon 7 differs by a C-to-T transition at position +6 of the *SMN1* exon 7 cDNA sequence. The amino acid sequence of the proteins deduced by the *SMN1* and *SMN2* cDNAs is the same and is shown under accession No. Q16637. The wildtype structure of *SMN1* and *SMN2* and useful mutant forms therof are also shown and described in the articles of C.L. Lorson et al. (1999), *supra,* and C.L. Lorson and E.J. Androphy (2000), *supra,* both references being incorporated herein by way of reference.

The substance according to the present invention may directly or indirectly control *in vivo* the inclusion of exon 7 of the *SMN2* gene. In the direct control, the substance may directly affect pre-mRNA processing or may directly interact with any one of the splicesome components involved in *SMN2* exon 7 splicing. Alternatively, since *SMN2* exon 7 splicing is regulated *in vivo* in a complex regulative system comprising specific kinase and phosphatase enzymes, the controlled generation, or the inhibition or the stimulation of these specific kinase and/or phosphatase enzymes are appropriate indirect control means for *SMN2* exon 7 splicing, consequently resulting in the prevention of exon 7 skipping or, in other terms, in the inclusion of exon 7. Included within the meaning of an indirect control is the expression of polypeptide factors through genetic engineering from their respective encoding polynucleotides (usually in the form of DNA, especially cDNA or genomic DNA), which polypeptide factors in turn interact with *SMN2* exon 7 splicing either directly or indirectly.

Embodiments for the substance which meet the above-mentioned features and objects will now be described in further detail.

A particularly suitable substance according to the present invention is a splicing regulator which specifically interacts with the splicing of exon 7 of the *SMN2* gene. Table 1 below shows a list of splicing regulators that may affect *SMN2* exon 7 splicing (only the name of the gene is given, while it is apparent that most genes have associated splice variants).

**Table 1**

| Factor | Source |
|---|---|
| SPp20 | Ayane, M. et al., Nucl. Acids Res. *19*, 1273-1278 (1991) |
| | |
| ASF/SF2 | Krainer, A.R. et al., Genes and Dev. *4,* 1158-1171 (1990) |
| | |
| SC35 | Fu, X.D. and Maniatis, T. Science *256,* 535-538 (1992) |
| | |
| SRp30c | Screaton, G.R. et al., EMBO J. *14,* 4336-4349 (1995) |
| | |
| SRp40 | Screaton, G.R. et al., EMBO J. *14,* 4336-4349 (1995) |
| | |
| SRp55 | Screaton, G.R. et al. EMBO J. *14,* 4336-4349 (1995) |
| | |
| SRp75 | Zahler, A.M. et al. Mol. Cell. Biol. *13,* 4023-4028 (1993) |
| | |
| 9G8 | Cavaloc, Y. et al. EMBO J. *13,* 2639-49 (1994) |
| Htra2-beta | Nayler, O. et al. Genomics 53, 191-202 (1998), and Beil, B. et al., DNA and Cell Biol. *16,* 679-690 (1997) |
| | |
| Htra2-alpha | Dauwalder, B. et al., Proc. Natl. Acad. Sci. USA *93*, 9004-9009 (1996) |
| | |
| SWAP | Sarkissian, M. et al., J. Biol. Chem. *271,* 31106-31114 (1996) |
| | |
| TASR | Yang, L. et al., J. Biol. Chem. 27761-27764 (1998) |
| | |
| PTB | Valcarcel, J. and Gebauer, F. Current Biol. *7*, R705-708 (1997) |
| | |
| SF-1 | Wang, X. et al., EMBO J. *18,* 4549-4559 (1999) |
| | |
| SLM-1 | Di Fruscio, M. et al. Proc. Natl. Acad. Sci. USA *96*, 2710-2715 (1999) |
| | |
| SLM-2 | Di Fruscio, M. et al., Proc. Natl. Acad. Sci. USA *96*, 2710-2715 (1999) |
| | |
| YT521 | Imai,Y. et al., Mol. Brain Res. 53, 33-40 (1998) |
| | |
| YT521-B | Hartmann, A.M. et al., Mol. Biol. Cell 10, 3909-3926 (1999) |
| | |
| U170K | Spritz, R.A. et al., Genomics *8*, 371-379 (1990) |
| | |
| U2AF65 | Zamore, P.D. et al., Nature *355*, 609-614 (1992) |
| | |
| U2AF35 | Zhang, M. et al., Proc. Natl. Acad. Sci. *89,* 8769-8773 (1992) |

Among the genes, or the corresponding polypeptides encoded thereby, being suitable for the present invention are also the fragments, derivatives or variants thereof which are effective as well for the prevention of *SMN2* exon 7 skipping.

To this end, the effect of several splicing regulatory factors were tested for their ability to stimulate FL-SMN2 expression in human cells *in vivo:* SF2/ASF, Htra2-betal, Htra2-beta3, SLM-2, YT521-B, SF1, U2AF65, PTB, CLK2, CLK-KR, hnRNPL and SRp30c. Among the 12 tested splicing regulatory factors Htra2-beta 1, an SR-like protein with Genebank Acc. No. U61267, concomitantly decreases the exon 7 skipped *SMN2* transcript and up-regulates full length *SMN2* expression, resulting in >90% full length *SMN2* transcript in human cells *in vivo.*

The splicing regulation observed in human cells is not species specific, as confirmed by similar *in vivo* splicing assays in murine cells. Co-transfection of increasing amounts of Htra2-betal (on the amino acid sequence level identical to the mouse Tra2 homolog) with *SMN2* minigene in mouse cells conferred high levels of full length *SMN2* expression and low levels of exon 7 skipped *SMN2*, comparable to the splicing pattern observed in human cells.

In the course of the investigations on splicing factors being suitable for the purpose of present invention, it was discovered that another splicing factor being tested, SF2/ASF (SRp30a), intensified the *SMN2* exon 7 skipping. Therefore, the inhibition of *SMN2* exon 7 specific splicing factors of this type leads to an enhanced inclusion of *SMN2* exon 7, thereby generating full length *SMN2* transcripts *in vivo* by an alternative mechanism as compared to, for example, the Htra2-betal splicing factor. The inhibition of the effect of SF2/ASF (SRp30a) or other splicing factors of this type can be achieved by inhibitory or antagonist substances and methods known to those skilled in the art, such as endogeneously expressing functionally ineffective variants, using oligonucleotide antisense probes or specific antibodies, or affecting the phosphorylation degree in order to inactivate the respective splicing factor by means of suitable substances as described below in more detail.

Alternatively, the substance according to the present invention is represented by a polynucleotide (DNA, especially cDNA or genomic DNA) which respectively encodes for the splicing regulator mentioned above having the function of preventing *SMN2* exon 7 skipping, such as the DNA which encodes for Htra2-beta1. Of course, DNAs are included which are defined by fragments, derivatives, alleles or mutant forms of the afore-mentioned DNAs, which fragments, derivatives, alleles or mutant forms are effective for preventing *SMN2* exon 7 skipping.

Instead of a splicing regulatory factor itself as explained above, it is also possible to make use of an upstream pathway regulatory factor or element which finally leads *in vivo* to the desired effect on the respective splicing regulator, thereby preventing *SMN2* exon 7 skipping.

As an example for such another embodiment of the present invention, the substance is a polypeptide which controls the phosphorylation or phosphorylation degree of a splicing regulator for the splicing of *SMN2* exon 7, or a DNA encoding such a polypeptide, including fragments, derivatives or mutant forms thereof being effective for controlling the phosphorylation degree of the *SMN2* exon 7 splicing regulator. A prefered example for such a phosphorylation controlling substance, which thereby indirectly controls *SMN2* exon 7 inclusion, is a kinase targeting on SR proteins or SR-like proteins. It may be assumed that phosphorylation of the SR protein-type splicing regulators results in a recruitment of those factors from intracellular storage pools, known as speckles. Phosphorylation may be controlled directly by means of kinases which phosphorylate the splicing regulators, such as SR protein kinases which are known per se, for example cd2-like SR protein kinases (types clkl-4) described by Nayler O. et al. (1997), Biochem. J. 326, 693-700. Conversely, instead of these kinase enzymes, phosphatases which specifically dephosphorylate an *SMN2* exon 7 splicing regulator may be suitable for stimulating or enhancing the inclusion of *SMN2* exon 7 as well. In this connection, the substance for controlling the phosphorylation of a splicing regulator, thereby controlling *SMN2* exon 7 inclusion, is preferably a SR protein phosphatase, or a DNA encoding therefor, including fragments, derivatives or mutant forms thereof being effective for controlling the phosphorylation degree of the *SMN2* exon 7 splicing regulator. While the cd2-like kinases (clkl-4) lead to the phosphorylation of the SR protein splicing factors, the corresponding phosphatases result in a de-phosphorylation of the clk targets. By means of clk-mediated phosphorylation or de-phosphorylation, the active concentration and/or phosphorylation degree of the SR protein targets can be changed and adjusted *in vivo,* thereby resulting in an indirectly controlled increase of exon 7 inclusion of the *SMN2* gene.

Further information on SR and SR-like proteins is provided in articles of Fu, X.D., RNA 1, 663-680 (1995) and Krämer A., Annu. Rev. Biochem. 65, 367-409 (1996). Furthermore, Cáceres J.F. et al., Science 265, 1706-1709 (1994), describe the antagonizing effect on the alternative splicing, including splice site selection and exon inclusion/skipping, between the SR protein SF2/ASF and the heterogeneous nuclear ribonucleoprotein (hnRNP), as studied by overexpression of the respective factors and its effect on the alternative splicing of the human β globin, the rat clathrin light chain B, the rat β-tropomyosin and the adenovirus E1A gene.

Since several such substances have been isolated for other systems, low molecular weight substances will exist which either stimulate or inhibit the afore-mentioned kinase or phosphatase enzymes. Accordingly, particularly suitable substances for the purpose of the present invention are inhibitors of a kinase or a phosphatase enzyme mentioned above. The phosphorylation degree of the afore-mentioned, *SMN2* exon 7-specific splicing regulators is in turn affected by these kinase and phosphatase inhibitors, consequently resulting again in the controlled prevention of exon 7 skipping of the *SMN2* gene.

Alternatively, the splicing factors affecting *SMN2* exon 7 processing may themselves be subject to activity-dependent regulation through *in vivo* alternative splicing patterns by means of low molecular weight substances. This is, for example, the case for the Htra2-beta factor, where the isoform structure and the isoform ratio of the factor, and thus the splicing activity can be regulated by fine tuning *in vivo* by means of the known cholinergic agonist pilocarpine (see R. Daoud et al. European Journal of Neuroscience, 11, 788-802 (1999)).

Furthermore, various hormones, for example steroid hormones and in particular sex steroid hormones like estrogens, have been shown to regulate alternative splicing (Guivarc'h, D. et al., Endocrinology 139, 4213-21 (1998)). Given the fact that rare cases of SMA, particularly females, show homozygous loss of *SMN1*, but no SMA phenotype (see Hahnen, E. et al., *Hum*. *Mol*. *Genet*. 4, 1927-1933 (1995); Cobben, J. et al., *Am. J*. *Hum. Genet*. 57, 805-808 (1995); and Wang, C.H., et al., *Hum*. *Mol*. *Genet* 5, 359-365 (1996)), it is contemplated that a sex specific factor may differentially regulate, possibly via the signal pathway targeting on the *SMN2* exon 7 specific splicing factors, the prevention of exon 7 skipping and thereby prevents them from SMA. Therefore, hormones and particularly sex-specific steroid hormones are excellent candidates as low molecular weight substances being suitable for the present invention.

For the medical treatment, the low molecular weight substances can be applied as therapeutic agents, in an effective amount to prevent exon 7 skipping of the *SMN2* gene, in appropriate pharmaceutical formulations comprising conventional additives.

When the DNAs described above are used as the substance according to the present invention, the mammalian cell is transfected in an appropriate transformation/transfection system known to those skilled in the art. The mammalian cells may be transiently transfected, but the respective DNA is preferably stably integrated into the genome of the cells. For this purpose, the mammalian cell may be exposed with the substance outside the body to be treated (*ex vivo).* As a suitable source, the mammalian cell may be a cell being extracted from the individual to be treated, a cell being isolated and brought into culture, or a suitable cell line. The above described DNA substances of the present invention may also be used in vectors which have been developed for gene therapy, such as the vectors for delivering therapeutic genes described by Mitani et al. in TIBTECH 11, 162-166 (1993). In such a vector, the DNA is usually operatively linked to an expression control DNA sequence known to those skilled in the art.

Accordingly, the present invention also provides a mammalian host cell which is stably transfected with a DNA, especially with a vector containing such DNA, where the DNA is as specified above.

The present invention also provides a process for changing the pre-mRNA processing relating to the *SMN* gene of a mammalian cell, which process comprises exposing the cell to a substance which is capable of controlling the inclusion of exon 7 of the *SMN2* gene. Again, the mammalian cell is preferably a human cell. By means of this process, the fidelity and the effectivity of a substance targeting on *SMN2* exon 7 skipping may be examined and observed by splicing analysis of the respective mammalian cells. Improved analysis is achieved if the mammalian cell to be exposed is stably transfected by the minigene constructs which will be described below in further detail.

The present invention also advantageously provides an efficient method for the testing of substances that prevent the skipping of exon 7 of the *SMN2* gene, which method comprises the steps of (1) exposing mammalian cells, which had been transfected with a DNA which comprises exon 7 of the *SMN2* gene or fragments or mutant forms thereof, to the substance to be tested, and (2) determining the ratio between exon 7 inclusion and exon 7 skipping of the *SMN2* gene as a result of exposure step (1). This method has been successfully applied for verifying the effectiveness of substances specified above for modifying *SMN2* exon 7 splicing, and it can also be advantageously used for finding further substances which may directly or indirectly prevent the skipping of *SMN2* exon 7. The test method according to the invention is particularly designed to be suitable for a high throughput screening for substances which putatively prevent the skipping of *SMN2* exon 7. The transfected cells may be incubated in plates having a high number of wells and treated with substances from a chemical library, especially substances having a relatively low molecular weight.

The mammalian cells, which are preferably human cells, are transfected with a DNA, which comprises exon 7 of the *SMN2* gene or fragments or mutant forms thereof, by means of a conventional method known to those skilled in the art.

In a preferred embodiment of the afore-mentioned screening method, the mammalian cells to be exposed to the tested substance had been transfected with a DNA which is in a form of a minigene construct. It has been found according to the present invention that both flanking exons 6 and 8 are important for exon 7 splicing. Therefore, not only minigene constructs containing *SMN2* exon 7 and at least partial sequences of the flanking introns upstream and downstream of exon 7, respectively, or mutant forms of the respective exon 7 and intron sequences, but minigene constructs which additionally contain exon 6 and exon 8, or fragments or mutant forms of these DNA sequences, are particularly useful for the test method according to the present invention. The minigene construct may further comprise at least one further foreign DNA element differing from the *SMN2* gene. For example, such a foreign DNA element(s) may serve as an internal splicing control. As another DNA element which is suitable for an internal control minigene construct, an exon 7 mutant form may be used which constitutively leads to exon 7 inclusion, including the *SMN1* exon 7 sequence. Furthermore, with other approproiate mutant forms of the minigene constructs, the responsive-element(s) on the SMN2 gene, which may be responsible for the prevention of exon 7 skipping, can be mapped more finely.

Importantly, splicing of *SMN* minigenes recapitulates splicing patterns of the respective endogenous *SMN* genes, especially when a cell to be used is stably transfected by the minigenes.

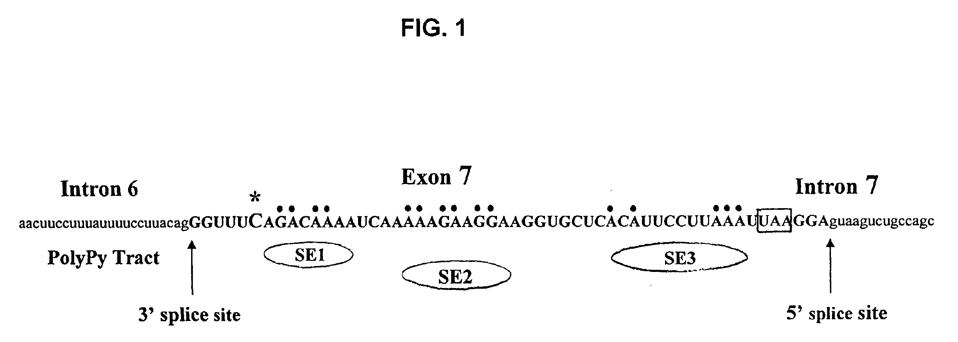

The RNA sequence of the *SMN* exon 7 region indicating exon 7 sequences (bold capital case) and adjacent portions of intronic sequences (lower case) is shown in Fig. 1 and listed as SEQ ID No. 1. Non-polymorphic *SMN1* and *SMN2* exon 7+6 position (large, bold and asterisk), splice sites, *SMN* stop codon (boxed), and mutations (dots) are indicated. As for the mutant forms, e.g. the base pairs indicated with dots can be mutated to "U" residues in the identified regions SE1, SE2 and SE3.

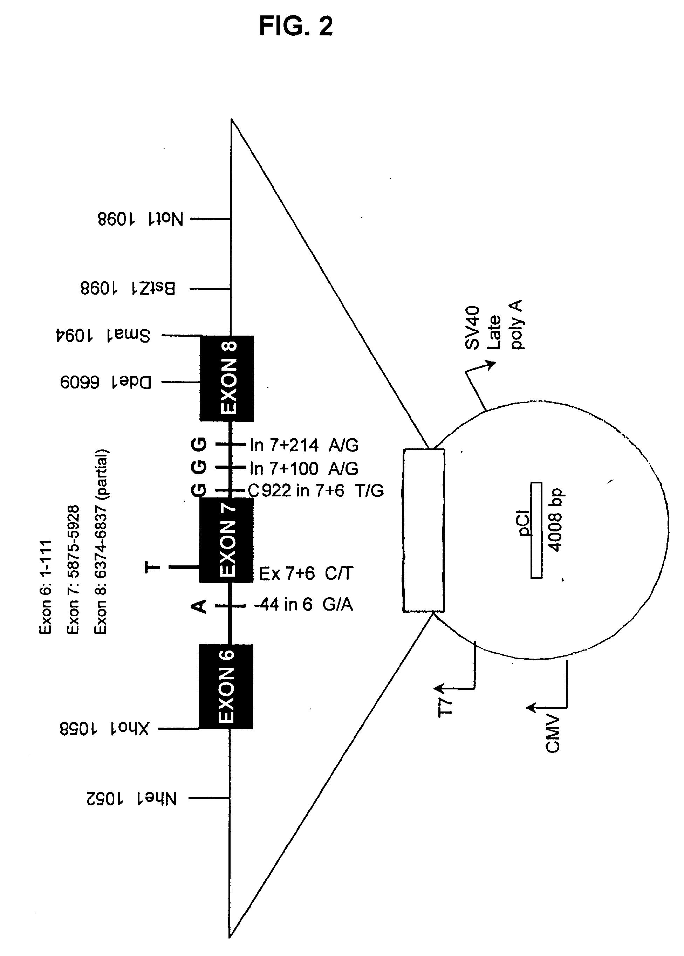

The wildtype SMN2 minigene construct useful for the present invention is schematically shown in Fig. 2 and described in more detail in Example 1 below.

When fragments of the flanking intron sequences are to be inserted into the minigene construct, relatively long fragments comprising the flanking upstream and downstream intron sequences of *SMN2* exon 7, respectively, are preferred.

The *SMN* minigene constructs may be produced by standard cloning methodology or by PCR cloning. Examples for suitable PCR primers to be used for wild-type and mutant constructs are described in Example 1 below. The afore-mentioned DNA elements should be fused into a plasmid or a vector suitable for transfecting the desired mammalian cells.

In the test method, after the transfected mammalian cells had been exposed to a substance which putatively prevents *SMN2* exon 7 skipping (step (1) mentioned above), the ratio between exon 7 inclusion and exon 7 skipping is determined (step (2)). Accordingly, the suitability and the effectiveness of the substance is checked. The *SMN2* exon 7 inclusion/skipping ratio may be determined as follows.

First, RNA obtained from the transfected and exposed cell is subjected to reverse transcription (RT). Then, the DNA obtained from reverse transcription is amplified such that exon 7 *SMN2* sequences of the *SMN2* gene may be amplified, if present, and the thus amplified DNA is subjected to DNA analysis which is capable of distinguishing between exon 7 inclusion and exon 7 skipping of the *SMN2* gene. Reverse transcription and the DNA amplification can be carried out by conventional methods, preferably by means of RT-PCR with suitable primer pairs. After the DNA fragments relevant for exon 7 inclusion or skipping have been amplified, an appropriate DNA analysis is performed which allows to distinguish between exon 7 inclusion and exon 7 skipping of the *SMN2* gene.

If a DNA analysis on a qualitative or semi-quantitative laboratory scale is desired and sufficient, conventional DNA electrophoresis may be conducted in order to separate and, thus, distinguish the differently sized, amplified fragments specific for either exon 7 inclusion or exon 7 skipping. The RT-PCR primer pair comprises a first primer being specific for either one of the respective exons adjacent to exon 7 (i.e. either one specific for exon 6 or one specific for exon 8), and a second primer being specific for the plasmid or vector sequence of the minigene construct and allowing primer extension spanning the exon 7 region in a direction reverse to that of the first primer. The generated DNA amplicons differing in size can be determined for example by DNA band staining. A more quantitative result can be obtained by conducting DNA analysis by at least two oligonucletide probes, one probe being specific for exon inclusion and one probe being specific for exon skipping. The probe which is specific for exon inclusion spans the *SMN2* exon 7 sequence of appropriate length, e.g. 15 to 25 nucleotides long, and the probe being specific for exon skipping spans the exon 6/exon 8 junction of the *SMN2* gene, i.e. covers a part of the 3'-end region of exon 6 and a part of the 5'- end region of exon 8 to provide an oligonucleotide probe of appropriate length, e.g. 15 to 25 nucleotides long.

In a preferred embodiment of the determination step (2), molecular beacon probes are used to provide an efficient and quantitative DNA analysis which can be carried out automatically. Molecular beacons are single-stranded nucleic acid molecules that possess a stem-and-loop structure (as described by Tyagi, S. and Kramer, F.R. in Nature Biotechnology 14, 303-308 (1996)). The loop portion of the molecule serves as a probe sequence that is complementary to the specific target nucleic acid. The stem is formed by the annealing of two complementary arm sequences that are on either side of the probe sequence. A fluorescent moiety is attached to the end of one arm, and a fluorescence quenching moiety is attached to the other arm. The stem hybrid keeps the fluorescer and the quencher so close to each other that fluorescence does not occur. When the molecular beacon probe encounters the specific target molecule, it forms a probe-target hybrid that is stronger and more stable than the stem hybrid. The probe then undergoes a spontaneous conformational re-organization that forces the arm sequence apart, separating the fluorophore from the quencher, and permitting the fluorophore to fluoresce. Accordingly, the molecular beacons may hybridize only to the specific target sequences that are perfectly complementary to the probe sequence, and the PCR reaction products can be analyzed and determined in a homogeneous assay in hermetically sealed glass capillaries or tubes, thereby avoiding contaminations. Using this method eliminates the need for gel electrophoresis and allows for automation. Using a pair of molecular beacon probes having differently colored fluorophors (see Tyagi, S. et al., Nature Biotechnology 16, 49-53 (1998)), the presence or absence of exon 7 in the RT-PCR amplicons obtained in step (2) of the present screening method can be determined simultaneously in one assay. That is, one molecular beacon probe is target-specific for the *SMN2* exon 7 sequence, and the other molecular beacon probe is target-specific for the *SMN2* exon 6/exon 8 junction. An optimized molecular beacon probe pair is the following, wherein the molecular beacon recognizing *SMN2* exon 7 inclusion is
TAMRA-cagcgcTAAGGAATGTGAGCACCTTCCgcgctg-6-FAM,
and the molecular beacon recognizing *SMN2* exon 7 skipping is
TAMRA-cgcgagcCCAGCATTTCCATATAATAGCCgctcgcg-TET.

In these examplified molecular beacon probes, the respective target-specific hybridizing sequence is shown in capital letters (SEQ ID Nos. 2 and 3), whereas the sequences shown in small letters correspond to the complementary stem part of the molecular beacon probe. Of course, the stem sequences can be replaced by appropriate alternative complementary stem portions. TAMRA (6-carboxy-tetramethylrodamine) denotes the moiety which quenches either one of the respective specific fluorophore moieties, 6-FAM (6-carboxy-fluorescein) and TET (tetrachloro-6-carboxy fluorescein). The fluorophore moieties which differ in their emitted fluorescence wavelength enables to distinguish between the two molecular beacon probes. Alternative to 6-FAM and TET, 2,7-dimethoxy-4, 5-dichlor-6-carboxyfluorescein (JOE), hexachloro-6-carboxyfluorescein (HEX), Texas Red, C3 or C5 may be used as fluorophore moieties as well. As a further alternative, a target-specific hybridizing sequence being complementary to the above specified molecular beacon probes may be used as well.

Instead of the molecular beacon approach, other known homogeneous PCR detection systems may be applied as well which are also suitable for being conducted in hermetically sealed glass capillaries or tubes, such as the TaqMan™ sytem or the LightCycler™. In the TaqMan™ sytem (described by Livak K.J. et al. in "PCR Methods Applic." 4, 357-362 (1995)), an oligonucleotide probe added to the PCR reaction is labeled at its ends with a fluorescing and a quenching moiety, respectively, thereby forming a quenched state. In the course of the PCR reaction the double labeled oligonucleotide is displaced from the template and at least partially digested. Thereby, the quenched state is diminished and the increase in fluorescence is an indication for the amount of amplicon generated during PCR. In the LightCycler™ system (see on internet under http://biochem.roche.com/lightcycler), two appropriately labeled oligonucleotide probes come in close proximity in a head to tail arrangement on the DNA template generated during PCR, thereby allowing fluorescence resonance energy transfer (FRET) to occur. The same fluorescing or quenching moieties as described above for the molecular beacon probes can be used for the TaqMan™ or the LightCycler™ sytem. In the commercial LightCycler™ sytem, the first oligonuclotide is labeled at its 3' end with fluorescein, whereas the second oligonucleotide carries another label (LC Red 640 or LC Red 705) at its 5' end.

The molecular beacon, the TaqMan™ and the LightCycler™ sytem are advantageous, because they allow, in sealed vessels or tubes free of contamination, a real time quantitative PCR determination of the ratio between exon 7 inclusion and exon 7 skipping in the test method according to the invention.

As a further product which is particularly suitable for the test method described above, the invention provides a mammalian host cell which is stably transfected with the minigene construct specified above, i.e. wherein minigene DNA elements are stably integrated within the genome of the mammalian host cell. A stable integration within the host cell genome is advantageous over transient transfection, because it provides more constant and reproducable results which can be standardized, i.e. characteristics being important for the screening test of the present invention.

The present invention will now be described in further detail by way of the following, non-limiting examples.

### Example 1

### Plasmid and minigene construction and mutant forms relating to SMN exon 7

Minigenes pSMN1 (wildtype), pSMN2 (wildtype) and derivative/mutant forms thereof contain genomic sequences from SMN exon 6 through exon 8 within the mammalian expression vector pCI (Promega) and were produced as previously described (Lorson, C. L., et al., Proc. Nat. Acad. Sci. USA 96, 6307-6311 (1999); Lorson, C. L. and Androphy, E., J *Hum*. *Mol*. *Genet.* Vol. 9, No. 2, 259-265 (2000)). These minigenes recapitulate the splicing pattern of the endogenous *SMN.* For pSMN1ex7^{Cen} a single nucleotide in exon7+6 had been exchanged and reveals a pSMN2-like splicing pattern in a pSMN1 background.

The vector construct of the *SMN2* widtype minigene is shown in Figs. 2. The nucleotide differences over the *SMN1* seuqence in this region are also indicated. For the construction of the *SMN* minigenes, the primers: 
Ex6FX (5'-CGATCTCGAGATAATTCCCCCACCACCTC-3')      SEQ ID No. 4
Ex8RS (5'-GCTACCCGGGCACATACGCCTCACATACA-3')      SEQ ID No. 5
 were used. The *pSMN1* minigene was also used as a template to generate oligo-mediated site-specific mutations within exon 7 and cloned into the appropriate plasmid backbone as described, thereby converting *SMN1* into *SMN2* minigenes. For example, for the *pSMN2* specific base pair exchange in exon 7 at position exon 7+6, the following primers were used (nucleotide changes are shown by small letters):

Forw. 5'-CTTACAGGGTTTcAGACAAAATC SEQ ID No. 6
Rev. 5'-GATTTTGTCTgAAACCCTGTAAG SEQ ID No. 7

The *SMN* minigenes were created by Expand High Fidelity (Roche Molecular) PCR Amplification of 250 ng of genomic DNA (Roche Molecular) by using the wildtype specific *SMN1* or *SMN2* primers defined above. Products were digested with *Xho*I and *Sma*I and cloned directionally into *Xho*I and *Sma*I sites within pCI and sequenced across the five nucleotide polymorphisms to identify *SMN1* and *SMN2* clones. Site-directed mutagenesis reactions with the primers listed above were performed by using Thermo Plot Vent polymerase (New England Biolabs).

### Example 2

### cDNA and vector constructions of SMN2 exon 7 specific splicing regulators

Tissue-specific splicing can be changed and analyzed under *in vivo* conditions with a minigene approach, following a protocol as described by O.Stoss et al., Brain Research Protocols 4, 383-394 (1999). The following splicing regulators were used.

### 1. Htra2-beta1

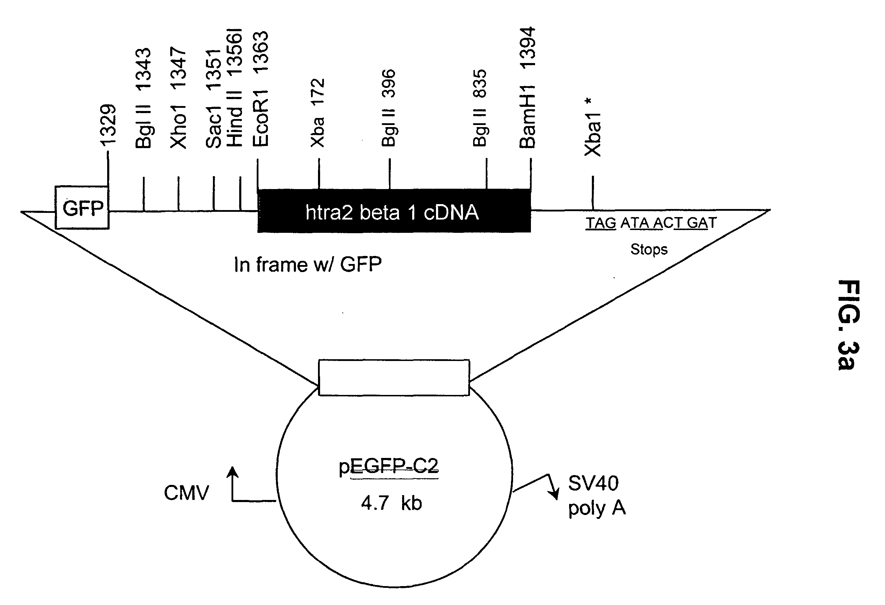

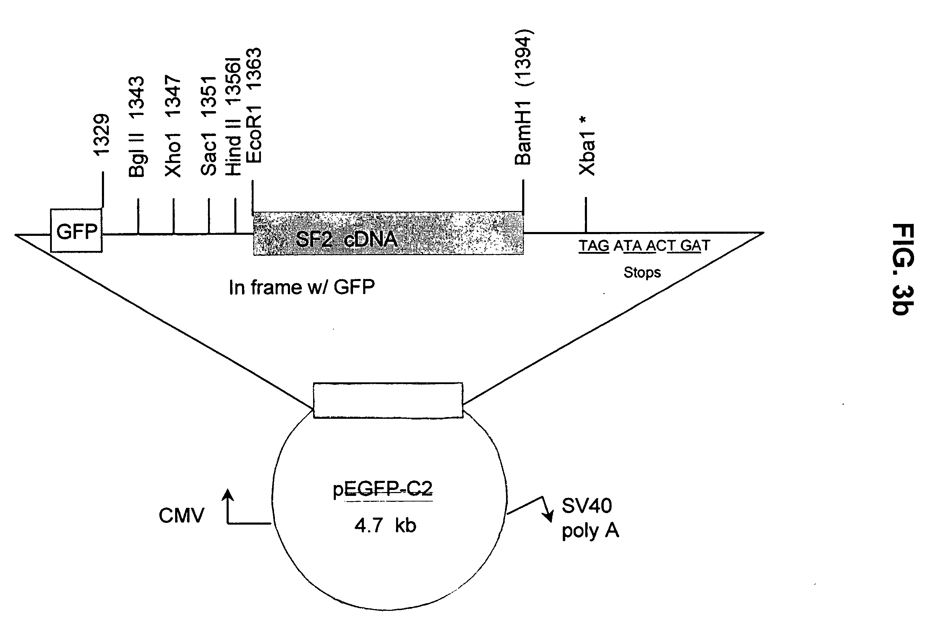

The cDNA for the coding region of Htra2-beta1 was obtained as an EcoRl-BamHl PCR-fragment and subcloned into the mammalian expression vector pEGFP-C2 (Clontech) to create a GFP-tagged fusion protein for transient transfection as described by Nayler, O., et al., *Genomics* 53, 191-202 (1998)). The transfection vector is schematically shown in Fig. 3 A.

### 2. SF2/ASF (SRp30a)

Analogous to Htra2-betal, the cDNA for the coding region of SF2/ASF (A.R. Krainer et al., Genes and Dev. 4, 1158-1171 (1990) was obtained as an EcoRl-BamHl PCR-fragment and likewise subcloned into the mammalian expression vector pEGFP-C2. The transfection vector is schematically shown in Fig. 3 B.

### Example 3

### Transfections of mammalian cells

Plasmid DNA was purified by cesium chloride banding (Sambrook et al., Molecular Cloning: A Laboratory Manual. 2^{nd} edition. Cold Spring Harbor Laboratory Press, Cold Spring Harbor, New York, 1989) or by using Quiagen columns (Qiagen, Hilden, Germany). The minigene constructs or vectors/plasmids obtained in Examples 1 and 2 were introduced into cells as follows.

Transient transfection of human HEK 293 cells was performed in 35 mm six-well dishes. The day before transfection 3x10⁵ cells were seeded and then either individually transfected, or co-transfected with a constant amount of *SMN* minigene construct (1µg) against a titration (0-3 µg) of *SMN2*-specific splicing factor (pEGFP-Htra2-betal or pEGFP-SF2) by standard calcium phosphate procedures. For titrated co-transfections, the appropriate amount of empty pEGFP-C2 vector was added to ensure that equal amounts of DNA were transfected. Mouse fibroblasts (NIH 3T3) were transiently transfected as described for the human cells, except for using Superfect transfection reagent (Qiagen) according to the manufacturer's protocol. Cells were incubated overnight at 37°C in 3% CO₂. The transfection efficiency could be monitored by fluorescence microscopy.

Stable transfection into the genome of a mammalian cell, typically a cell line, can be carried out as follows: The SMN2 minigene construct obtained in Example 1 is subcloned into a pTARGET vector (Promega). This vector is designed for stable transfection; it allows stable expression through selection by means of neomycin resistance (G418) and a fast screening method for positive clones by means of a blue/white selection, as performed according to the manufacturer's instructions. On the other hand, the *SMN2*-specific splicing factor cDNA, for example a plasmid described in Example 2, is subcloned into the pHygEGFP vector (Clontech) containing a hygromycin resistance. This vector allows a selection of stably transfected cells and a visual selection of positive clones. Cultivation and selection is again performed according to the manufacturer's instructions. The clones which show the best matching with the transcription pattern of the endogeneous *SMN2* gene are finally selected.

### Example 4

### Changing and analyzing in vivo splicing of the SMN2 gene

### 1) Prevention of exon 7 skipping of the SMN2 gene

24 h (HEK 193 cells) or 36 h (NIH 3T3 cells) after transient co-transfection with a constant amount of *SMN2* wildtype minigene construct (see Example 1; 1µg) and increasing amounts (0-3 µg) of *SMN2*-specific splicing factor (pEGFP-Htra2-betal, see Example 2) in accordance with the procedure described in Example 3, total RNA was isolated from equally transfected cultures using the E.Z.N.A. total RNA kit (Peqlab). Reverse transcription (RT-) PCR was performed using the SMN exon 8 specific reverse primer (5 pmol/µl) (SMNex8-rev: 5' GCC TCA CCA CCG TGC TGG; SEQ ID No. 8), 5x first strand buffer (Life Technologies), 100 mM DTT, 10 mM dNTP, 50 U Superscript II RT (Life Technologies) and 7 U RNase inhibitor (Pharmacia). To ensure that only plasmid-derived SMN transcript was detected, subsequent amplification was performed with a vector-specific forward primer (pCI-forw: 5' GGT GTC CAC TCC CAG TTC AA; SEQ ID No. 9) and the *SMN* specific reverse primer SMNex8-rev. This was necessary since all cell lines contain endogenous *SMN*. Following 30 cycles of amplification with annealing and extension temperatures of 55°C for 30 s and 72°C for 1 min, PCR products were resolved on a 2% agarose gel, containing ethidium bromide.

However, in order to allow simultaneous amplificiation of an internal standard derived from HPRT, reverse transcription using oligo-dT primer was also performed. Subsequent semi-quantitative multiplex PCR was carried out with pCI-forw, SMNex8-rev primers and highly conserved HPRT-primers

(Fw 5`-AAG GAG ATG GGA GGC CAT, and
Rev 5`-GTT GAG AGA TCA TCT CCA CCA AT) (SEQ ID Nos. 10 and 11).

The ratio of FL-SMN to SMNΔ7 was densitometrically determined using the One-DScan software (MWG-Biotech). All experiments were verified numerous times.

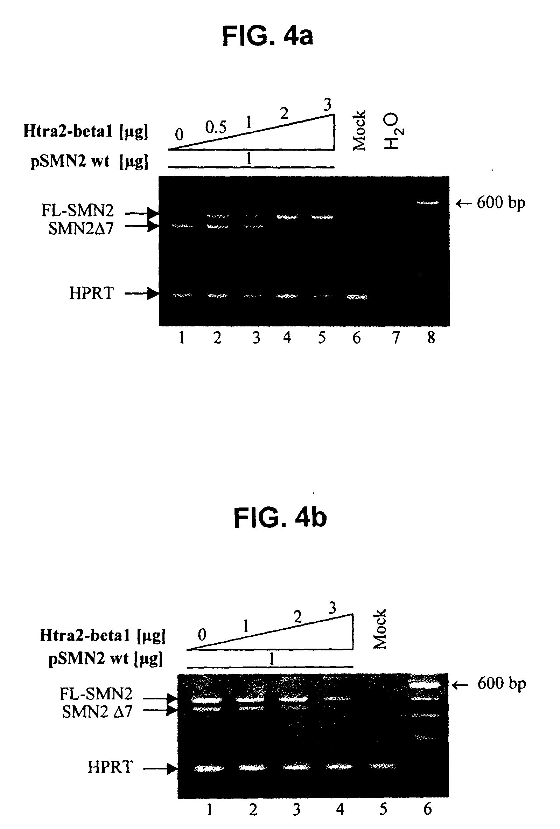

The results of the stimulation of full length *SMN2* expression by means of co-transfection and co-expression with the *SMN2* exon 7 specific splicing factor Htra2-betal is shown in Fig. 4, wherein Fig. 4A shows the results obtained in human cells and Fig. 4B shows the results obtained in mouse cells. pSMN2wt recapitulates the splicing pattern of the endogenous *SMN2*, which reveals a ratio of 40% FL-SMN2 to 60% *SMN2Δ7* in human cells (lane 1 of Fig. 4A) and of 68% *FL-SMN2* to 32% *SMN2Δ7* in mouse cells (lane 1 of Fig. 4B). Over-expression of Htra2-betal led to abundant accumulation of FL-*SMN2* in a concentration-dependant manner, as shown in lanes 2 to 5, where the level of FL-*SMN2* increases to 78% (lane 4) and 90% (lane 5) in human cells (Fig. 4A), and to 82% (lane 3), and up to 89% (lane 4) in mouse cells (Fig. 4B).

Accordingly, it is established that functionally active SMN product can be generated in vivo by means of the substance according to the present invention which prevents the skipping of exon 7 of the *SMN2* gene.

### 2) Modulation of SMN in vivo splicing depending on SMN minigene mutant forms

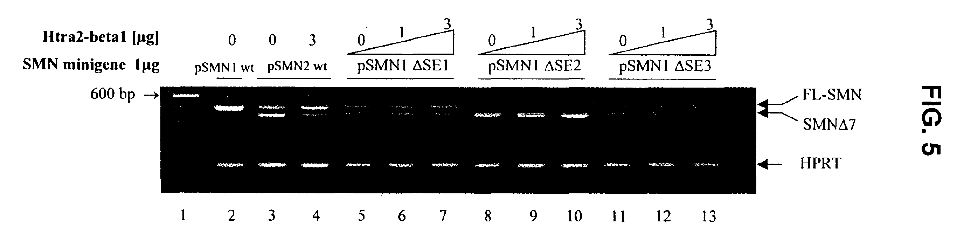

The procedure of the above step 1) was repeated in the same manner, except that various mutant forms of the SMN minigenes described in Example 1 was used instead of the *SMN2* wildtype minigene construct. The results are shown in Fig. 5. The transcription pattern of *pSMN1wt* and *pSMN2wt* minigenes are given in lane 2-4 of Fig. 5 for easy comparison to *SMN1* mutated minigenes.

*SMN1* minigenes containing mutations within SE1 or SE3 showed an increased level of *SMN1Δ7* in the absence of Htra2-betal (lane 5, 11 ). In each instance, co-transfection of Htra2-betal restored partially high levels of FL-*SMN* (lane 6, 7, 12 and 13). Disruption of the AG-rich element SE2 resulted only in *SMN1Δ7* transcript and could not be overcome even at the highest concentration of Htra2-betal (lane 8-10).

Thus, the fidelity and the effectivity of a substance targeting on *SMN2* exon 7 skipping can be examined and verified by splicing analysis using mammalian cells being transfected by the minigene constructs according to the present invention.

### 3) Purification of recombinant proteins effective for SMN2 exon 7 skipping

GST-tagged Htra2-betal was purified from BL21(DE3)pLysS *E*.*coli* transformed with pGEX-3X-Htra2-betal and induced with 1mM IPTG at 37°C for 2 h. Cells were harvested by centrifugation, washed, resuspended in 5 ml NETN-500 (20 mM Tris at pH 7.5, 500 mM NaCl, 1 mM EDTA, 0.5 % NP-40, 4 mM NaF) and sonicated for 3 x 15 sec. Supernatants were bound to glutathione-agarose beads, washed 4 times in NETN-500 and then eluted with glutathione-elution buffer (10 mM glutathione, 50 mM Tris at pH 7.5).

Htra2-betal protein binds to *SMN2* exon 7 exonic splice enhancer sequences, as confirmed by further *in vitro* experiments directed to UV-mediated cross-linking of cellular factors and *SMN* exon 7 RNA.

### 4) Phosphorylation of recombinant proteins effective for SMN2 exon 7 skipping

GST-Htra2-betal fusion protein was phosphorylated by incubation in HeLa S100 extract under splicing conditions with 0.5 mM ATP, 4 mM creatine phosphate, 2 mM MgCl₂, 20 mM KCl at 30°C for 45 min according to the method described by Xiao, S. H. and Manley, J. L. (Genes Dev. 11, 334-344 (1997). Mock phosphorylation was performed without addition of ATP, creatine phosphate and MgCl₂. All phosphorylated and mock-phosphorylated proteins were repurified by binding to glutathione-agarose beads followed by extensive washing with NETN-500. Protein concentrations were determined by BioRad DC Protein assay kit and confirmed by visualizing ~1 µg of the fusion protein following SDS-PAGE analysis and Coomassie blue staining.

### Example 5

### Changing in vivo splicing of the SMN2 gene in an alternative manner

Transient transfection of HEK293 cells was performed in 35 mm six-well dishes. 3x10⁵ cells were co-transfected with 1µg *SMN2* wildtype minigene and increasing amounts of pEGFP-SF2 vector DNA by standard calcium phosphate procedures. The appropriate amount of empty pEGFP-C2 vector was added to ensure that equal amounts of DNA were transfected.

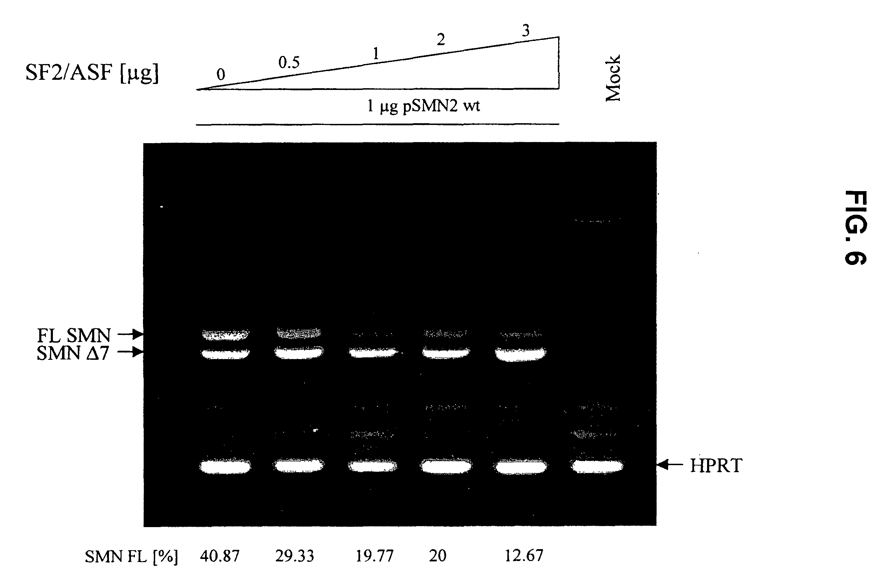

24 h after co-transfection, total RNA was isolated, and RT-PCR analysis was performed as described in Example 4. The effects of SF2/ASF expression on the generation of full length SMN2 transcripts is shown in Fig. 6. Accordingly, increasing amounts of effective SF2/ASF decreases FL *SMN2* and enhances *SMN2* exon 7 skipping. These results establish that decreasing the active amount or inhibiting the effect of splicing factors, which naturally enhance *SMN2* exon 7 skipping, results in an increased level of FL *SMN2* and thus alternatively prevents exon 7 skipping.

As a result, substances which are capable of preventing the skipping or exclusion of exon 7 of the SMN gene are provided by the present invention and described herein. The substances can reverse "wrong" SMN2 splicing pattern, thus generating full length transcripts and thus functionally active SMN protein on the basis of the SMN2 gene *in vivo.* Since "wrong" SMN2 splicing is associated with disorder or disease states causing spinal muscular atrophy, but SMN2 genes still exist in viably individuals, the substances can be used as therapeutic agents. The invention also provides a method for testing substances that may control exon 7 inclusion of the SMN2 gene. The method is suitable for a high throughput screening.
Substances which are capable of preventing the skipping or exclusion of exon 7 of the SMN gene are described. The substances can reverse "wrong" SMN2 splicing pattern, thus generating full length transcripts and thus functionally active SMN protein on the basis of the SMN2 gene *in vivo.* Since "wrong" SMN2 splicing is associated with disorder or disease states causing spinal muscular atrophy, but SMN2 genes still exist in viably individuals, the substances can be used as therapeutic agents. The invention also provides a method for testing substances that may control exon 7 inclusion of the SMN2 gene. The method is suitable for a high throughput screening.

## Claims

1. A substance which is capable of preventing the skipping of exon 7 of the *SMN2* gene for use as a therapeutic agent.

2. The substance according to claim 1 which is a splicing regulator for the splicing of exon 7 of the *SMN2* gene.

3. The substance according to claim 1 or 2 which is the polypeptide denoted by Htra2-beta1, or fragments, derivatives, or variants of this polypeptide being effective for preventing the skipping of exon 7 of the *SMN2* gene.

4. The substance according to claim 1 or 2 which is a DNA encoding for Htra2-betal, or fragments, derivatives, alleles or mutant forms of said DNA, wherein said fragments, derivatives, alleles or mutant forms are effective for preventing the skipping of exon 7 of the *SMN2* gene.

5. The substance being a DNA as defined in claim 4, wherein the respective DNA is used for transfection of mammalian cells for being used as a therapeutic agent.

6. The substance according to claim 5, wherein the DNA is inserted into a vector which is suitable for transfecting mammalian cells.

7. The substance according to claim 1 which is a steroid hormone.

8. The substance according to any one of claims 1 to 7 for use in the treatment of spinal muscular atrophy (SMA).

9. A process for changing the pre-mRNA processing relating to the *SMN* gene of a mammalian cell, which process comprises exposing the cell to a substance which is capable of controlling the inclusion of exon 7 of the *SMN2* gene.

10. The process according to claim 9, wherein a substance as defined in any one of claims 1 to 7 is used for exposure to said mammalian cell.

11. A method for testing substances that may control the inclusion of exon 7 of the *SMN2* gene, comprising the steps of:
(1) exposing a mammalian cell, which is transfected with a DNA which comprises exon 7 of the *SMN2* gene or fragments or mutant forms thereof, to the substance to be tested, and
(2) determining the ratio between exon 7 inclusion and exon 7 skipping as a result of exposure step (1).

12. The method according to claim 11, wherein the mammalian cell is transfected by a minigene construct comprising the following DNA elements derived from the *SMN2* gene:
• exon 7 or fragments or mutant forms thereof,
• each flanking intron sequence upstream and downstream of exon 7, respectively, or fragments or mutant forms thereof, and
• exon 6 and exon 8, or fragments or mutant forms thereof.

13. The method according to claim 11 or 12, wherein said DNA elements are fused into a plasmid or a vector.

14. The method according to any one of claims 11 to 13, wherein the mammalian cell is stably transfected.

15. The method according to claim 11, wherein step (2) includes a step of subjecting the RNA obtained from the exposed cells to reverse transcription, a step of amplifying the DNA obtained from reverse transcription such that exon 7 sequences of the *SMN2* gene may be amplified if present, and a step of performing a DNA analysis distinguishing between exon 7 inclusion and exon 7 skipping.

16. The method according to claim 15, wherein the DNA analysis is conducted by at least two probes, one probe being specific for exon 7 inclusion and one one probe being specific for exon 7 skipping.

17. The method according to claim 15 or 16, wherein the DNA analysis is conducted using molecular beacon probes, or using oligonucleotide probe pairs which allow fluorescence resonance energy transfer to occur on the analyzed DNA.

18. The method according to claim 16 or 17, wherein molecular beacon probes are used, the probe specific for exon 7 inclusion comprising an oligonucleotide sequence shown in SEQ ID NO. 2 or its complementary sequence, and the probe specific for exon 7 skipping comprising an oligonucleotide sequence shown in SEQ ID NO. 3 or its complementary sequence.

19. A mammalian host cell which is stably transfected with a DNA being selected from the group of DNAs encoding polypeptides which are capable of at least partially preventing the skipping of exon 7 of the *SMN2* gene.

20. A mammalian host cell according to claim 19, wherein said DNA encodes for a splicing regulator for the splicing of exon 7 of the *SMN2* gene.

21. The substance according to claim 20, wherein the DNA encodes for Htra2-betal, or fragments, derivatives, alleles or mutant forms of said DNA, wherein said fragments, derivatives, alleles or mutant forms are effective for preventing the skipping of exon 7 of the *SMN2* gene.

22. A mammalian host cell which is stably transfected with a minigene construct comprising the following DNA elements derived from the *SMN2* gene:
• exon 7 or fragments or mutant forms thereof,
• each flanking intron sequence upstream and downstream of exon 7, respectively, or fragments or mutant forms thereof, and
• exon 6 and exon 8, or fragments or mutant forms thereof.

23. The mammalian host cell according to any one of claims 19 to 22, wherein said DNA is fused into a plasmid or a vector.


SEQUENCE LISTING

<110> Max-Planck-Gesellschaft zur Förderung der Wissensc

<120> Substances for the Treatment of Spinal Muscular Atrophy

<130> EP 26076

<140>
<141>

<160> 11

<170> PatentIn Ver. 2.1

<210> 1
<211> 91
<212> RNA
<213> Artificial Sequence

<220>
<223> Description of Artificial Sequence: SMN1 exon 7
      with 5' and 3' proximal intron junctions

<400> 1
aacuuccuuu auuuuccuua caggguuuca gacaaaauca aaaagaagga aggugcucac 60
auuccuuaaa uuaaggagua agucugccag c                               91

<210> 2
<211> 21
<212> DNA
<213> Artificial Sequence

<220>
<223> Description of Artificial Sequence:
      Oligonucleotide

<400> 2
taaggaatgt gagcaccttc c                                          21

<210> 3
<211> 22
<212> DNA
<213> Artificial Sequence

<220>
<223> Description of Artificial Sequence:
      Oligonucleotide

<400> 3
ccagcatttc catataatag cc                                              22

<210> 4
<211> 29
<212> DNA
<213> Artificial Sequence

<220>
<223> Description of Artificial Sequence:
      Oligonucleotide

<400> 4
cgatctcgag ataattcccc caccacctc                                       29

<210> 5
<211> 29
<212> DNA
<213> Artificial Sequence

<220>
<223> Description of Artificial Sequence:
      Oligonucleotide

<400> 5
gctacccggg cacatacgcc tcacataca                                       29

<210> 6
<211> 23
<212> DNA
<213> Artificial Sequence

<220>
<223> Description of Artificial Sequence:
      Oligonucleotide

<400> 6
cttacagggt ttcagacaaa atc                                             23

<210> 7
<211> 23
<212> DNA
<213> Artificial Sequence

<220>
<223> Description of Artificial Sequence:
      Oligonucleotide

<400> 7
gattttgtct gaaaccctgt aag                                              23

<210> 8
<211> 18
<212> DNA
<213> Artificial Sequence

<220>
<223> Description of Artificial Sequence:
      Oligonucleotide

<400> 8
gcctcaccac cgtgctgg                                                    18

<210> 9
<211> 20
<212> DNA
<213> Artificial Sequence

<220>
<223> Description of Artificial Sequence:
      Oligonucleotide

<400> 9
ggtgtccact cccagttcaa                                                  20

<210> 10
<211> 18
<212> DNA
<213> Artificial Sequence

<220>
<223> Description of Artificial Sequence:
      Oligonucleotide

<400> 10
aaggagatgg gaggccat                                                    18

<210> 11
<211> 23
<212> DNA
<213> Artificial Sequence

<220>
<223> Description of Artificial Sequence:
      Oligonucleotide

<400> 11
gttgagagat catctccacc aat                                                23